## Europäisches Patentamt

## European Patent Office

## Office européen des brevets

(11) Publication number: **0 124 018**
**B1**

## EUROPEAN PATENT SPECIFICATION

(45) Date of publication of patent specification: **25.11.87**

(51) Int. Cl.⁴: **A 61 K 37/02, A 61 K 35/16**

(21) Application number: **84104360.7**

(22) Date of filing: **17.04.84**

(54) **Pharmaceutical preparation containing purified fibronectin.**

(30) Priority: **28.04.83 US 489624**

(43) Date of publication of application:
**07.11.84 Bulletin 84/45**

(45) Publication of the grant of the patent:
**25.11.87 Bulletin 87/48**

(84) Designated Contracting States:
**AT BE CH DE FR GB IT LI LU NL SE**

(56) References cited:
**EP-A-0 035 204**
**EP-A-0 047 216**

(73) Proprietor: **Armour Pharmaceutical Company**
**303 South Broadway**
**Tarrytown New York 10591 (US)**

(72) Inventor: **Landaburu, Ricardo H.**
**1 Magnolia Drive**
**Rye Town New York (US)**
Inventor: **Yue, Robert H.**
**259-15 86th Avenue**
**Floral Park New York (US)**
Inventor: **Farb, David L.**
**Toms Way Road**
**LaGrangeville New York (US)**
Inventor: **Violand, Bernard N.**
**15553 Summer Lake Drive**
**Chesterfield Missouri (US)**

(74) Representative: **Patentanwälte Grünecker, Dr.**
**Kinkeldey, Dr. Stockmair, Dr. Schumann, Jakob,**
**Dr. Bezold, Meister, Hilgers, Dr. Meyer-Plath**
**Maximilianstrasse 58**
**D-8000 München 22 (DE)**

Courier Press, Leamington Spa, England.

# 0 124 018

**Description**

This invention relates to a lyophilized fibronectin preparation prepared from a solution containing 1—16 mg/ml fibronectin and 0.01% to 0.4% w/v of a pharmaceutically acceptable surface active agent. The stable freeze-dried solid composition containing fibronectin can be readily reconstituted.

Fibronectin is a large complex protein possessing important biological activities as a structural component of tissues and as a protein modulating and controlling the function of cells in the body in ways that range from normal growth to repair of damage and participation in the defense against invading foreign substances and organisms. It is known by a variety of names that reflect its diverse biological activities including the names of: large external transformation sensitive protein (LETS); cell surface protein (CSP); cell adhesion protein (CAP); opsonic $\alpha_2$ surface binding glycoprotein; and cold insoluble globulin (CIG). Pharmacological application of fibronectin includes treatment of septic shock and treatment of infective diseases. Due to its action of enhancing intercellular adhesive properties and its effect on the morphology of cancer cells, fibronectin is a potential candidate for cancer treatment.

Fibronectin is obtained from fractions of plasma protein or fibroblast culture fluid, purified and pasteurized to eliminate the risk of contamination by viruses, and formulated for clinical application.

To be suitable for clinical application, fibronectin must be in a readily injectible or infusable form, must possess a high degree of therapeutic activity, must be stable on storage prior to use and must be homogeneous at the time of injection without aggregated particles or precipitates therein.

Purified fibronectin obtained by many techniques of purification common to the art is unstable in liquid media, undergoing progressive molecular decomposition on prolonged storage unless it is stored in the frozen state. The decomposition is accelerated by room and elevated temperatures resulting in hydrolysis and formulation into discrete fragments of the polypeptide structure. The loss of therapeutic activity due to decomposition is substantial even within a few weeks rendering the composition ineffective for the intended purpose. Instability may be due to the presence of trace quantities of a proteolytic enzyme which may not be completely removed in the purification of the fibronectin. To stabilize fibronectin and prevent loss of therapeutic activity therein, fibronectin preparations are lyophilized for storage and reconstituted prior to injection or infusion into the patient. While lyophilization has been found effective in preventing the undesirable decomposition, it created a problem with reconstitution: fibronectin at low temperatures precipitates, hence the name cold insoluble globulin, and on reconstitution in aqueous media a portion of fibronectin remains precipitated requiring extended time periods for dissolution. This is undesirable to both patient and personnel administering the fibronectin, since time is of considerable importance for proper treatment of various conditions. Also, it is left to chance that health care personnel will uniformly insure complete dissolution of fibronectin in each vial containing unit dosages prior to administration to patients.

It is known to use stabilizing agents in protein preparations in order to prevent denaturation or other alteration of the proteins. Such stabilizers include albumin, amino acids, gelatin, glycine, and various sugars. It is also known that some of these stabilizing agents enhance the solubility and help prevent aggregation of some freeze dried proteins upon reconstitution.

EP—A—35204 and EP—A—47216 disclose compositions comprising fibronectin and surface active agents.

While such agents are used to advantage in certain protein formulations, they do not always lend a solution to the problems associated with freeze dried fibronectin preparations, namely, problems of stability, therapeutic activity and reconstitution.

Therefore, it is the object of the present invention to provide a stable lyophilized fibronectin preparation. Said object is achieved by a lyophilized fibronectin preparation prepared from an aqueous solution containing 1—16 mg/ml fibronectin and 0.01% to 0.4% w/v of a pharmaceutically acceptable surface active agent characterized in that the solution further contains 2% to 10% w/v of a monosaccharide, a disaccharide, mannitol or sorbitol.

For intravenous administration the preparation is in the form of an aqueous solution.

Additionally, a neutral salt such as sodium chloride is added to adjust osmolar concentration and a buffer salt is used to control pH of the media. The solution may be of 0.002 M to 0.05 M with regard to the buffer salt, which may be sodium citrate, sodium glycinate, sodium phosphate, tris(hydroxymethyl)aminomethane or mixtures thereof. The preferred buffer salt is sodium citrate, which in small quantities enhances solubility of fibronectin and also possesses mild chelating properties helping to prevent fibronectin polymerization.

In general, the purified and pasteurized fibronectin in an aqueous solution containing the above-described ingredients is lyophilized into a solid in suitable containers and stored. Prior to use, the solid is reconstituted with sterile water.

The active component of the formulations of the present invention in an aqueous solution is purified and pasteurized fibronectin comprising about 85 to 100% of the dissolved proteinaceous solids which can be obtained from human plasma, cryoprecipitate fraction of human plasma, a sub-fraction of the cryoprecipitate of human plasma, and Fraction I and Fraction I—0 of human plasma. The procedure for obtaining the purified fibronectin may be any of the procedures used by the prior art that result in such purification. The purified and pasteurized fibronectin so obtained is present in an aqueous solution

2

containing about 1 to about 16 mg/ml and preferably about 3 to about 8 mg/ml of fibronectin. Generally, such aqueous solutions also contain a buffer and/or neutral salt in sufficient quantities to maintain solubility of fibronectin and to regulate the pH from about 6 to 8, preferably from about 6.5 to 7.5 and most preferably from 6.8 to 7.3.

In practicing the present invention it is desirable to so design the final steps of the purification and pasteurization of fibronectin that the ingredients used therein are the same as, or are compatible with, the ingredients of the formulations of the present invention. This desired result may also be accomplished by exchange or removal of certain components, such as buffer or neutral salts by the technique of dialysis across a semi-permeable membrane or diafiltration. Alternatively, permeation chromatography technique on hydrophilic gel media, such as cross-linked dextran, agarose, or polyacrylamide may be employed for removal of certain components from the fibronectin solution.

As previously noted, highly purified solutions of fibronectin in which fibronectin comprises about 85 to 100% and preferably at least 95% of the dissolved proteinaceous solids undergo molecular decomposition on extended storage in liquid media. The decomposition is progressive with time and is accelerated by room and elevated temperatures. While the mechanism of decomposition is not well understood, the resultant preparation containing fragments of fibronectin is not well-suited for clinical use. Lyophilization on the other hand, prevents such decomposition and is therefore an effective means for preserving the biological properties of fibronectin. Table I illustrates the typical results obtained on analysis of the respective solutions.

TABLE I
Decomposition of fibronectin with time at 20°C

|  | After 1 wk. | After 1 mo. | After 3 mos. |
|---|---|---|---|
| Aqueous solution of fibronectin | 5% | 30% | 100% |
| Lyophilized fibronectin | 0 | 0 | 0 |

It can be readily ascertained from the above results that lyophilization prevents decomposition of fibronectin on storage. However, upon trying to reconstitute the lyophilized fibronectin great difficulty was experienced with the dissolution of fibronectin as shown in the following comparative examples.

Comparative Example 1

Compositions of fibronectin (Fn) containing 189 mg of fibronectin, in a buffer of 0.13 M NaCl, 0.05 M glycine sodium glycinate, 0.01 M sodium citrate, at pH 7.3, having amounts of albumin indicated herein were lyophilized and then reconstituted with 50 ml of water. Percent dissolution of fibronectin at various time intervals are shown. The amount of fibronectin dissolved was determined by absorbance measurement at 280 nm using an A280 of 12.8 for 1% (w/v) fibronectin solution.

**0 124 018**

| Albumin | Time in minutes after recon. | % of Fn in solution |
|---------|------------------------------|---------------------|
| 0 | 15 | 53.3 |
| | 30 | 63.1 |
| | 60 | 64.7 |
| | 1440 | 82.2 |
| 2 mg/ml | 15 | 59.2 |
| | 30 | 76.4 |
| | 60 | 82.0 |
| | 1440 | 105.0 |
| 5 mg/ml | 15 | 64.5 |
| | 30 | 75.6 |
| | 60 | 78.5 |
| | 1440 | 90.2 |
| 10 mg/ml | 15 | 69.0 |
| | 30 | 79.3 |
| | 60 | 82.5 |
| | 1440 | 95.5 |
| 20 mg/ml | 15 | 73.2 |
| | 30 | 78.0 |
| | 60 | 78.2 |
| | 1440 | 89.0 |

Without albumin, even after 1440 minutes, only 82.2% of the fibronectin is dissolved.

Although albumin enhances dissolution of fibronectin, the indicated time intervals for dissolution are too long to be of practical value.

Comparative Example 2

Compositions of fibronectin containing 189 mg of fibronectin, in a buffer of 0.08 M NaCl, 0.01 M citrate, at pH 7.3, having 2.5% w/v sucrose and amounts of albumin indicated herein were lyophilized and then reconstituted with 50 ml of water. Percent dissolution of fibronectin at various time intervals are shown. The amount of fibronectin dissolved was determined as in Example 1.

| Albumin | Time in minutes after recon. | % of Fn in solution |
|---------|------------------------------|---------------------|
| 0 | 15 | 47.7 |
| | 30 | 47.7 |
| | 60 | 48.8 |
| | 1440 | 73.0 |
| 2 mg/ml | 15 | 87.8 |
| | 30 | 93.1 |
| | 60 | 94.4 |
| | 1440 | 105.0 |
| 5 mg/ml | 15 | 79.8 |
| | 30 | 85.1 |
| | 60 | 85.9 |
| | 1440 | 100.9 |
| 10 mg/ml | 15 | 82.0 |
| | 30 | 90.5 |
| | 60 | 90.7 |
| | 1440 | 108.6 |
| 20 mg/ml | 15 | 83.0 |
| | 30 | 90.5 |
| | 60 | 92.0 |
| | 1440 | 105.0 |

4

With albumin and sucrose present there appears to be certain improvement in enhancement of dissolution, however, the required dissolution time is still impractical.

Comparative Example 3

Compositions of fibronectin containing 125 mg of fibronectin, in a buffer of 0.075 M NaCl, 0.002 M citrate, at pH 7.2, having amounts of surfactant Tween® 80 indicated herein were lyophilized and then reconstituted with 25 ml of water. Percent dissolution of fibronectin at various time intervals are shown. The amount of fibronectin dissolved was determined as in Example 1.

| % of Tween® 80 | Time in minutes after recon. | % of Fn in solution |
|---|---|---|
| 0.02 | 15 | 48.4 |
| | 30 | 57.8 |
| | 60 | 75.8 |
| 0.05 | 15 | 49.2 |
| | 30 | 66.4 |
| | 60 | 82.0 |
| 0.10 | 15 | 58.6 |
| | 30 | 70.4 |
| | 60 | 83.6 |
| 0.20 | 15 | 47.6 |
| | 30 | 58.6 |
| | 60 | 81.2 |

The surfactant used in this example is not effective in enhancing dissolution of fibronectin.

Comparative Example 4

Compositions of fibronectin containing 189 mg of fibronectin, in a buffer of 0.08 M NaCl, 0.01 M citrate at pH 7.3, having 2.5% w/v glucose and amounts of the surfactant Pluronic® F68 indicated herein were lyophilized and then reconstituted with 50 ml of water. Percent dissolution of fibronectin at various time intervals are shown. The amount of fibronectin dissolved was determined as in Example 1.

| % of Pluronic® F68 | Time in min. after recon. | % of Fn in solution |
|---|---|---|
| 0.02 | 15 | 84.9 |
| | 30 | 79.8 |
| | 60 | 79.3 |
| | 1440 | 103.3 |
| 0.05 | 15 | 103.2 |
| | 30 | 105.6 |
| | 60 | 107.2 |
| | 1440 | 126.1 |
| 0.10 | 15 | 68.7 |
| | 30 | 67.4 |
| | 60 | 67.1 |
| | 1440 | 82.8 |
| 0.40 | 15 | 93.2 |
| | 30 | 98.5 |
| | 60 | 99.4 |
| | 1440 | 103.0 |

dissilution of fibronectin in the presence of Pluronic® F68 and glucose is enhanced.

We have discovered that selected ingredients used in lyophilized formulations containing fibronectin enables full and complete solution of fibronectin in a short period of time, ranging from a couple to 20 minutes, preferably to less than 10 minutes, and most preferably to less than one minute. The reconstituted solution of fibronectin prepared in accordance with the present invention is safe and effective and may be injected or infused into patients in need of fibronectin.

A preferred embodiment of the invention comprises:

a., an aqueous solution which contains 1—16 mg/ml, preferably about 3—8 mg/ml of fibronectin;

b., is 0.002 M to 0.05 M, preferably 0.005 M to 0.02 M with regard to a physiologically acceptable buffer salt selected from sodium citrate, sodium glycinate, sodium phosphate, and tris(hydroxymethyl)aminomethane or mixtures thereof;

c., is 0.03 M to 0.09 M, and preferably 0.04 to 0.08 M with regard to a neutral salt, such as sodium chloride, to adjust the osmolar concentration of the formula to that of the physiologic milieu of about 0.14 M of sodium chloride.

d., contains 2% to 10% w/v, and preferably about 3% to 7% w/v of a monosaccharide, or a disaccharide such as glucose, galactose, mannose, sucrose, lactose, maltose or mannitol, or sorbitol; and

e., contains 0.01% to 0.4% w/v, preferably 0.02% to 0.1% w/v of a pharmaceutically acceptable surface active agent selected from: poly(oxyalkylenes) mono- and tri-sorbitan esters (fatty acid esters of sorbitol and its anhydrides copolymerized with a varying number of moles of ethylene oxide) such as Polysorbate 80 (an oleate ester), Polysorbate 20 (a laurate ester), Polysorbate 40 (a palmitate ester), and Polysorbate 60 (a stearate ester); alkyl phenyl polyoxyethylenes (such as Triton® and Nomidet®); anionic agents such as bile salts (sodium taurocholate, sodium cholate, sodium deoxycholate and sodium glycocholate); and polyhydric alcohols with surface active properties such as the high molecular weight copolymers of propylene glycol and propylene oxide sold under the trade names of Pluronic® F-38 and Pluronic® F-68.

The above described ingredients are admixed and the pH of the mixture is adjusted, using an acid or a base, to the value of 6.5—7.5 and preferably 6.8—7.3. The mixture is then lyophilized in suitable containers for storage.

The following examples illustrate the preparations of the present invention.

Example 1

To 13 liters of an aqueous solution containing about 7 mg/ml of fibronectin, 0.01 M sodium citrate and 0.075 M NaCl at pH 6.7 was added 0.5 kg of parental grade glucose and 10 grams of Polysorbate 80 (PS 80). The solution was gently stirred until all components dissolved. The solution was then diluted to 20 liters with an aqueous buffer consisting of 0.01 M sodium citrate, 0.075 M NaCl at about pH 7.0. The pH of the resultant solution was verified to be between 6.8—7.3, then the solution was subjected to initial clarification on a non-sterile filter and then sterilized by passage through a sterile, bacterially retentive filter. The sterile solution was then aseptically dispensed into glass vials, 50 ml per vial, fitted with an appropriate rubber stopper of the lyophilizing type. The vials were placed in a freezer, and after freezing, subjected to lyophilization by the procedure customarily used in the art.

Example 2

To about 14 liters of an aqueous solution containing about 8.2 mg/ml of fibronectin, 6% w/v of sucrose, 0.005 M sodium citrate, 0.005 M sodium glycinate at a pH of 6.5 was added 135 g of NaCl, and 7.5 g of Polysorbate 80. After dissolution of the ingredients by stirring, the volume was adjusted to 15 liters with an aqueous buffer solution of 0.005 M of sodium citrate and 0.005 M of sodium glycinate at pH 7.2. The pH of the diluted solution was adjusted with dilute HCl and NaOH and was verified to be between 6.8—7.3. The solution was then further processed as described in Example 1.

Example 3

Eight liters of an aqueous solution containing 7.6 mg/ml of purified fibronectin, 0.001 M sodium citrate, and 0.16 M NaCl at pH 7.0 was diluted two-fold with an aqueous buffer consisting of 0.01 M sodium citrate, 5.0% w/v glucose and 8 grams of Pluronic® F-68 at pH 7.0. After stirring to obtain complete dissolution the pH of the resultant solution was between 6.8—7.3. The solution was then further processed as described in Example 1.

Example 4

An aqueous solution of 12 mg/ml fibronectin was obtained containing 8 M urea and 0.01 M sodium citrate at pH 7.2 from a commercial source. The solution was processed over a chromatographic column of Sephadex® G-25 equilibrated with an aqueous buffer solution containing 5.0% w/v sucrose, 0.05 M NaCl, and 0.02 M sodium citrate at pH 7.0. During chromatographic equilibration of two 3 liter batches of purified fibronectin on a column bed of 16 liters the fibronectin was diluted from the initial concentration of 12 mg/ml to about 8 mg/ml. The two batches of purified fibronectin were then pooled. Nine liters of this pooled solution was further diluted with additional column buffer to obtain a fibronectin concentration of about 6 mg/ml. To 12 liters of the diluted solution was added with stirring 6.0 g of Polysorbate 80. The pH of the resultant solution was between 6.8—7.3. The solution was further processed as described in Example 1.

Example 5

To 5 liters of an aqueous solution containing 6.3 mg/ml purified fibronectin, 0.075 M NaCl, 0.002 M sodium citrate at pH 7.2 was added 0.325 kg of parenteral grade maltose and 5.0 g of Polysorbate-80. The ingredients were dissolved by gentle stirring. The solution was diluted to about 6.5 liters with an aqueous solution containing 0.01 M sodium citrate, 0.075 M NaCl at pH 7.0. The pH of the resultant solution was adjusted to 6.8—7.3 using dilute HCl and NaOH. The solution was further processed as described in Example 1.

Example 6

To 6.3 liters of an aqueous solution containing 8.1 mg/ml of purified fibronectin, 0.002 M sodium citrate and 0.05 M NaCl at pH 7.2 was added 0.5 kg of parenteral grade sucrose and 5 g of sodium taurocholate. After mixing to dissolve the ingredients, the volume was adjusted to 10.0 liters with an aqueous solution containing 0.05 M NaCl and 0.002 M sodium citrate at pH 7.2. The solution was then further processed as described in Example 1.

Example 7

To 5 liters of an aqueous solution containing 6.2 mg/ml of purified fibronectin, 0.002 M sodium citrate, and 0.05 M NaCl at pH 7.1 was added 0.30 kg of parenteral grade sucrose and 3.07 g of Pluronic® F-68. The solution was mixed to dissolve the ingredients and the volume was adjusted to 6.1 liters with an aqueous solution containing 0.05 M NaCl and 0.002 M sodium citrate at pH 7.1. The pH of the solution was adjusted to 6.8—7.3 using dilute HCl and NaOH. The solution was then further processed as described in Example 1.

Compositions of the final formulas are presented in Table II.

TABLE II

| Formulas | 1 | 2 | 3 | 4 | 5 | 6 | 7 |
|---|---|---|---|---|---|---|---|
| Fibronectin | 4.5 mg/ml | 7.6 mg/ml | 3.8 mg/ml | 5.5 mg/ml | 5.1 mg/ml | 5.1 mg/ml | 5.1 mg/ml |
| Buffer salt | 0.01 M Citrate | 0.005 M Citrate 0.005 M Glycine | 0.01 M Citrate | 0.02 M Citrate | 0.002 M Citrate | 0.002 M Citrate | 0.002 M Citrate |
| Neutral Salt (NaCl) | 0.075 M | .075 M | 0.08 M | 0.05 M | 0.075 M | 0.05 M | 0.05 M |
| Carbohydrate | 2.5% Glucose | 5.0% Sucrose | 5.0% Glucose | 5.0% Sucrose | 5.0% Maltose | 5.0% Sucrose | 5.0% Sucrose |
| Surfactant | PS-80 0.05% | PS-80 0.05% | Pluronic® F-68 0.05% | PS-80 0.10% | PS-80 0.05% | Tauro-cholate 0.05% | Pluronic® F-68 0.05% |

**0 124 018**

The following example describes the dosage form for human administration.

Example 8

Quantitative composition of human fibronectin

| Ingredient | Amount per container |
|---|---|
| Fibronectin, Human | 202.5 mg—275 mg |
| Sucrose | 2.5 gm±0.25 |
| Sodium Chloride | 0.146 gm±0.015 |
| Polysorbate 80 | 0.025 gm±0.003 |
| Sodium Citrate | 0.028 gm±0.003 |
| Water for Injection | 50 ml* |

\* Removed during lyophilization.

The lyophilized formulations of the present invention are easily reconstituted prior to use. Illustrative data on reconstitution is presented in Example 9.

Example 9

Fibronectin lyophilized in the presence of 0.075 M NaCl, 0.002 M sodium citrate and containing the herein denoted amounts of carbohydrates and surfactants were reconstituted with 48.5 ml of water. Reconstitution time and remarks are shown hereinbelow.

| Carbohydrate | Surfactant | Recon. time | Remarks |
|---|---|---|---|
| 5% sucrose | — | — | A large clump floating, 79% soluble after 60 min. |
| 5% sucrose | 0.05% Tween® 80 | 3 min. 45 sec. | Clear no particulate |
| 5% sucrose | 0.05% Pluronic® F68 | 5 min. 15 sec. | Clear no particulate |
| 5% sucrose | 0.05% Taurocholate | 6 min. 45 sec. | Clear no particulate |
| 2.5% glucose | — | — | A large clump floating, 90% soluble after 60 min. |
| 2.5% glucose | 0.05% Tween® 80 | 16 min. 30 sec. | A few very small particles. |
| 2.5% glucose | 0.05% Pluronic® F68 | 10 min. 15 sec. | A few very small particles |
| 2.5% glucose | 0.05% Taurocholate | 34 min. 30 sec. | A few very small particles |

The results in Example 9 clearly show the unexpected and greatly improved solubility within short time periods of the lyophilized fibronectin in the formulations of the present invention.

**Claims for the Contracting States: BE, CH, DE, FR, GB, IT, LI, LU, NL, SE**

1. A lyophilized fibronectin preparation prepared from an aqueous solution containing:
1—16 mg/ml fibronectin; and
0.01% to 0.4% w/v of a pharmaceutically acceptable surface active agent characterized in that the solution further contains 2% to 10% w/v of a monosaccharide a disaccharide, mannitol or sorbitol.

2. The preparation of claim 1 wherein the solution further is 0.03 M to 0.09 M with regard to a neutral salt.

3. The preparation of claim 2 wherein the neutral salt is sodium chloride.

9

4. The preparation of claim 1 wherein the solution further is 0.002 M to 0.05 M with regard to a physiologically acceptable buffer salt.

5. The preparation of any of claims 1 to 4 wherein the buffer salt is sodium citrate, sodium glycinate, sodium phosphate, tris(hydroxymethyl)aminomethane, or mixtures thereof.

6. The preparation of any of claims 1 to 5 wherein the mono- or di-saccharide is glucose, galactose, mannose, sucrose, lactose or maltose.

7. The preparation of any of claims 1 to 6 wherein the surface active agent is: polyoxy ethylene sorbitan mono- and tri-esters; alkyl phenyl polyoxyethylenes; sodium taurocholate, sodium cholate, sodium deoxycholate, sodium glycocholate; or polyhydric alcohols.

8. The preparation of claim 7 wherein the polyoxy ethylene sorbitan ester contains the fatty acid oleate, laurate, stearate or palmitate.

9. The preparation of any of claims 1 to 8 wherein the preparation should be reconstituted with sterile water.

10. The preparation of any of claims 1 to 7 wherein the preparation contains on reconstitution with sterile water:

1—16 mg/ml of fibronectin;

is 0.002 M to 0.05 M with regard to a physiologically acceptable buffer salt selected from sodium citrate, sodium glycinate, sodium phosphate or tris(hydroxymethyl)aminomethane;

is 0.03 M to 0.09 M with regard to sodium chloride;

contains 2% to 10% w/v of glucose, galactose, mannose, sucrose, lactose, maltose, mannitol or sorbitol; and

contains 0.01% to 0.4% w/v of a pharmaceutically acceptable surface active agent selected from polyoxy ethylene sorbitan mono- and tri- esters; alkyl phenyl polyoxyethylenes; sodium taurocholate, sodium cholate, sodium deoxycholate, sodium glycocholate; or polyhydric alcohols.

11. The preparation of claims 1 to 7 wherein the preparation contains on reconstitution with sterile water:

3—8 mg/ml fibronectin;

is 0.005 M to 0.02 M with regard to a physiologically acceptable salt selected from sodium citrate, sodium glycinate, sodium phosphate or tris(hydroxymethyl)aminomethane;

is 0.04 M to 0.08 M with regard to sodium chloride;

contains 3% to 7% w/v of glucose, galactose, mannose, sucrose, lactose, maltose, mannitol, or sorbitol; and

contains 0.02% w/v to 0.1% w/v of a pharmaceutically acceptable surface active agent selected from polyoxy ethylene sorbitan mono- and tri-esters; alkyl phenyl polyoxyethylenes; sodium taurocholate, sodium cholate, sodium deoxycholate, sodium glycocholate; or polyhydric alcohols.

12. The preparation of any of claims 9 to 11 wherein the preparation has a pH of 6.5 to 7.5.

13. The preparation of claim 10 wherein said polyoxy ethylene sorbitan ester contains the fatty acid oleate, laurate, stearate and palmitate.

14. The fibronectin preparation of claim 10 wherein said lyophilized fibronectin is reconstituted into an aqueous solution within 20 minutes.

**Claims for the Contracting State: AT**

1. A process for preparing a lyophilized fibronectin preparation by lyophilizing an aqueous solution containing 1—16 mg/ml fibronectin and 0.01% to 0.4% w/v of a pharmaceutically acceptable surface active agent characterized in that the solution further contains 2% to 10% w/v of a monosaccharide, a disaccharide, mannitol or sorbitol.

2. The process of claim 1 characterised in that the solution is 0.03 M to 0.09 M with regard to a neutral salt.

3. The process of claim 2 characterised in that the neutral salt is sodium chloride.

4. The process of claim 1 characterised in that the solution is 0.002 M to 0.05 M with regard to a physiologically acceptable buffer salt.

5. The process of any of claims 1 to 4 characterised in that the buffer salt is sodium citrate, sodium glycinate, sodium phosphate, tris(hydroxymethyl) aminoethane, or mixtures thereof.

6. The process of any of claims 1 to 5 characterised in that the mono- or disaccharide is glucose, galactose, mannose, sucrose, lactose or maltose.

7. The process of any of claims 1 to 6 characterised in that the surface active agent is: polyoxy ethylene sorbitan mono- and tri-esters; alkyl phenyl polyoxyethylenes; sodium taurocholate, sodium cholate, sodium deoxycholate, sodium glycocholate; or polyhydric alcohols.

8. The process of claim 7 characterised in that the polyoxy ethylene sorbitan ester contains the fatty acid oleate, laurate, stearate or palmitate.

9. The process of any of claims 1 to 8 characterised in that the preparation is reconstituted with sterile water.

10. The process of any of claims 1 to 7 characterised in that the preparation contains on reconstitution with sterile water:

1—16 mg/ml of fibronectin;

is 0.002 M to 0.05 M with regard to a physiologically acceptable buffer salt selected from sodium citrate, sodium glycinate, sodium phosphate or tris(hydroxymethyl)amino methane;

is 0.03 M to 0.09 M with regard to sodium chloride;

contains 2% to 10% w/v of glucose, galactose, mannose, sucrose, lactose, maltose, mannitol or sorbitol; and

contains 0.01% to 0.4% w/v of a pharmaceutically acceptable surface active agent selected from polyoxy ethylene sorbitan mono- and tri-esters; alkyl phenyl polyoxyethylenes; sodium taurocholate, sodium cholate, sodium deoxycholate, sodium glycocholate; or polyhydric alcohols.

11. The process of any of claims 1 to 7 characterised in that the preparation contains on reconstitution with sterile water:

3—8 mg/ml fibronectin;

is 0.005 M to 0.02 M with regard to a physiologically acceptable salt selected from sodium citrate, sodium glycinate, sodium phosphate or tris(hydroxymethyl)aminomethane;

is 0.04 M to 0.08 M with regard to sodium chloride;

contains 3% to 7% w/v of glucose, galactose, mannose, sucrose, lactose, maltose, mannitol, or sorbitol; and

contains 0.02% w/v to 0.1% w/v of a pharmaceutically acceptable surface active agent selected from polyoxy ethylene sorbitan mono- and tri-esters; alkyl phenyl polyoxyethylenes; sodium taurocholate, sodium cholate, sodium deoxycholate, sodium glycocholate; or polyhydric alcohols.

12. The process of any of claims 9 to 11 characterised in that the preparation has a pH of 6.5 to 7.5.

13. The process of claim 10 characterised in that said polyoxy ethylene sorbitan ester contains the fatty acid oleate, laurate, stearate and palmitate.

14. The process of claim 10 characterised in that said lyophilized fibronectin is reconstituted into an aqueous solution within 20 minutes.

**Patentansprüche für die Vertragsstaaten: BE, CH, DE, FR, GB, IT, LI, LU, NL und SE**

1. Lyophilisierte Fibronektinherstellung, hergestellt aus einer wässrigen Lösung, enthaltend 1—16 mg/ml Fibronektin und 0,01% bis 0,4% in Gew./Vol. eines pharmazeutisch annehmbaren oberflächenaktiven Mittels, dadurch gekennzeichnet, daß die Lösung weiterhin 2% bis 10% in Gew./Vol. eines Monosaccharids, eines Disaccharids, Mannitol oder Sorbitol enthält.

2. Herstellung nach Anspruch 1, worin die Lösung weiterhin 0,03 M bis 0,09 M bezüglich eines neutralen Salzes ist.

3. Herstellung nach Anspruch 2, worin das neutrale Salz Natriumchlorid ist.

4. Herstellung nach Anspruch 1, worin die Lösung weiterhin 0,002 M bis 0,05 M ist bezuglich eines physiologisch annehmbaren Puffersalzes.

5. Herstellung nach einem der Ansprüche 1 bis 4, worin das Puffersalz Natriumcitrat, Natriumglycinat, Natriumphosphat, Tris(hydroxymethyl)aminomethan oder Mischungen daraus ist.

6. Herstellung nach einem der Ansprüche 1 bis 5, worin das Mono- oder Disaccharid Glucose, Galactose, Mannose, Sucrose, Lactose oder Maltose ist.

7. Herstellung nach einem der Ansprüche 1 bis 6, worin das oberflächenaktive Mittel Polyoxyethylensorbitanmono- und -triester, Alkylphenylpolyoxyethylene, Natriumtaurocholat, Natriumcholat, Natriumdeoxycholat, Natriumglycocholat oder mehrwertige Alkohole ist.

8. Herstellung nach Anspruch 7, worin der Polyoxyethylensorbitanester eine Fettsäure als Oleat, Laurat, Stearat oder Palmitat enthält.

9. Herstellung nach einem der Ansprüche 1 bis 8, worin die Herstellung mit sterilem Wasser rekonstituiert sein sollte.

10. Herstellung nach einem der Ansprüche 1 bis 7, worin die Herstellung nach der Rekonstitution mit sterilem Wasser 1—16 mg/ml Fibronektin enthält, 0,002 M bis 0,05 M bezüglich eines physiologisch annehmbaren Puffersalzes, gewählt aus Natriumcitrat, Natriumglycinat, Natriumphosphat oder Tris(hydroxymethyl)aminomethan, ist, 0,03 M bis 0,09 M bezüglich Natriumchlorid ist, 2% bis 10% in Gew./Vol. Glucose, Galactose, Mannose, Sucrose, Lactose, Maltose, Mannitol oder Sorbitol enthält und 0,01% bis 0,4% in Gew./Vol. eines pharmazeutisch annehmbaren oberflächenaktiven Mittels, gewählt aus Polyoxyethylensorbitanmono- und -triestern, Alkylphenylpolyoxyethylenen, Natriumtaurocholat, Natriumcholat, Natriumdeoxycholat, Natriumglycocholat oder mehrwertigen Alkoholen, enthält.

11. Herstellung nach den Ansprüchen 1 bis 7, worin die Herstellung nach der Rekonstitution mit sterilem Wasser 3 bis 8 mg/ml Fibronektin enthält, 0,005 M bis 0,02 M bezüglich eines physiologisch annehmbaren Salzes, gewählt aus Natriumcitrat, Natriumglycinat, Natriumphosphat oder Tris(hydroxymethyl)aminomethan, ist,

0,04 M bis 0,08 M bezüglich Natriumchlorid ist, 3% bis 7% in Gew./Vol. Glucose, Galactose, Mannose, Sucrose, Lactose, Maltose, Mannitol oder Sorbitol enthält und

0,02% bis 0,1% in Gew./Vol. eines pharmazeutisch annehmbaren oberflächenaktiven Mittels, gewählt aus Polyoxyethylensorbitanmono- und -triestern, Alkylphenylpolyoxyethylenen, Natriumtaurocholat, Natriumcholat, Natriumdeoxycholat, Natriumglycocholat oder mehrwertigen Alkoholen, enthält.

12. Herstellung nach einem der Ansprüche 9 bis 11, worin die Herstellung einen pH von 6,5 bis 7,5 besitzt.

13. Herstellung nach Anspruch 10, worin der Polyoxyethylensorbitanester Fettsäure als Oleat, Laurat, Stearat und Palmitat enthält.

14. Herstellung nach Anspruch 10, worin das lyophilisierte Fibronektin inceiner wässrigen Lösung innerhalb von 20 Minuten rekonstituiert wird.

**Patentansprüche für den Vertragsstaat: AT**

1. Verfahren zur Herstellung einer lyophilisierten Fibronektinherstellung durch Lyophilisieren einer wässrigen Lösung, enthaltend 1—16 mg/ml Fibronektin und 0,01% bis 0,4% in Gew./Vol. eines pharmazeutisch annehmbaren oberflächenaktiven Mittels,
dadurch gekennzeichnet,
daß die Lösung weiterhin 2% bis 10% in Gew./Vol. eines Monosaccharids, eines Disaccharids, Mannitol oder Sorbitol enthält.

2. Verfahren nach Anspruch 1, dadurch gekennzeichnet, daß die Lösung 0,03 M bis 0,09 M bezüglich eines neutralen Salzes ist.

3. Verfahren nach Anspruch 2, dadurch gekennzeichnet, daß das neutrale Salz Natriumchlorid ist.

4. Verfahren nach Anspruch 1, dadurch gekennzeichnet, daß die Lösung 0,002 M bis 0,05 M bezüglich eines physiologisch annehmbaren Puffersalzes ist.

5. Verfahren nach einem der Ansprüche 1 bis 4, dadurch gekennzeichnet, daß das Puffersalz Natriumcitrat, Natriumglycinat, Natriumphosphat, Tris(hydroxymethyl)aminomethan oder eine Mischung daraus ist.

6. Verfahren nach einem der Ansprüche 1 bis 5, dadurch gekennzeichnet, daß das Mono- oder Disaccharid Glucose, Galactose, Mannose, Sucrose, Lactose oder Maltose ist.

7. Verfahren nach einem der Ansprüche 1 bis 6, dadurch gekennzeichnet, daß das oberflächenaktive Mittel Polyoxyethylensorbitanmono- und -triester, Alkylphenylpolyoxyethylene, Natriumtaurocholat, Natriumcholat, Natriumdeoxycholat, Natriumglycocholat oder mehrwertige Alkohole ist.

8. Verfahren nach Anspruch 7, dadurch gekennzeichnet, daß der Polyoxyethylensorbitanester eine Fettsäure als Oleat, Laurat, Stearat oder Palmitat enthält.

9. Verfahren nach einem der Ansprüche 1 bis 8, dadurch gekennzeichnet, daß die Herstellung mit sterilem Wasser rekonstituiert ist.

10. Verfahren nach einem der Ansprüche 1 bis 7, dadurch gekennzeichnet, daß die Herstellung nach der Rekonstitution mit sterilem Wasser
1—16 mg/ml Fibronektin enthält,
0,002 M bis 0,05 M bezüglich eines physiologisch annehmbaren Puffersalzes, gewählt aus Natriumcitrat, Natriumglycinat, Natriumphosphat oder Tris(hydroxymethyl)aminomethan, ist,
0,03 M bis 0,09 M bezüglich Natriumchlorid ist, 2% bis 10% in Gew./Vol. Glucose, Galactose, Mannose, Sucrose, Lactose, Maltose, Mannitol oder Sorbitol enthält, und
0,01% bis 0,4% in Gew./Vol. eines pharmazeutisch annehmbaren oberflächenaktiven Mittels, gewählt aus Polyoxyethylensorbitanmono- und -triestern, Alkylphenylpolyoxyethylenen, Natriumtaurocholat, Natriumcholat, Natriumdeoxycholat, Natriumglycocholat oder mehrwertigen Alkoholen, enthält.

11. Verfahren nach einem der Ansprüche 1 bis 7, dadurch gekennzeichnet, daß die Herstellung nach der Rekonstitution mit sterilem Wasser
3—8 mg/ml Fibronektin enthält,
0,005 M bis 0,02 M bezüglich eines physiologisch annehmbaren Salzes, gewählt aus Natriumcitrat, Natriumglycinat, Natriumphosphat oder Tris(hydroxymethyl)aminomethan, ist,
0,04 M bis 0,08 M bezüglich Natriumchlorid ist, 3% bis 7% in Gew./Vol. Glucose, Galactose, Mannose, Sucrose, Lactose, Maltose, Mannitol oder Sorbitol enthält und
0,02% bis 0,1% in Gew./Vol. eines pharmazeutisch annehmbaren oberflächenaktiven Mittels, gewählt aus Polyoxyethylensorbitanmono- und -triestern, Alkylphenylpolyoxyethylenen, Natriumtaurocholat, Natriumcholat, Natriumdeoxycholat, Natriumglycocholat oder mehrwertigen Alkoholen, enthält.

12. Verfahren nach einem der Ansprüche 9 bis 11, dadurch gekennzeichnet, daß die Herstellung einen pH von 6,5 bis 7,5 besitzt.

13. Verfahren nach Anspruch 10, dadurch gekennzeichnet, daß der Polyoxyethylensorbitanester eine Fettsäure als Oleat, Laurat, Stearat und Palmitat enthält.

14. Verfahren nach Anspruch 10, dadurch gekennzeichnet, daß das lyophilisierte Fibronektin in einer wässrigen Lösung innerhalb von 20 Minuten rekonstituiert wird.

**0 124 018**

**Revendications pour les Etats Contractants: BE, CH, DE, FR, GB, IT, LU, NL, SE**

1. Préparation de fibronectine lyophilisée préparée en partant d'une solution aqueuse contenant: 1 à 16 mg/ml de fibronectine et 0,01 à 0,4% en poids/volume d'un surfactif acceptable pharmaceutiquement, caractérisée par le fait que la solution contient en outre 2 à 10% en poids/volume d'un monosaccharide, d'un disaccharide, de mannitol ou de sorbitol.

2. Préparation selon la revendication 1, caractérisée par le fait que la solution est 0,03 M à 0,09 M en ce qui concerne un sel neutre.

3. Préparation selon la revendication 2, caractérisée par le fait que le sel neutre est le chlorure de sodium.

4. Préparation selon la revendication 1, caractérisée par le fait que la solution est en outre 0,002 M à 0,05 M en ce qui concerne un sel tampon physiologiquement acceptable.

5. Préparation selon l'une quelconque des revendications 1 à 4, caractérisée par le fait que le sel tampon est le citrate de sodium, le glycinate de sodium, le phosphate de sodium, le tris-(hydroxyméthyl)-aminométhane ou des mélanges de ceux-ci.

6. Préparation selon l'une quelconque des revendications 1 à 5, caractérisée par le fait que le mono- ou disaccharide est le glucose, le galactose, le mannose, le sucrose, le lactose ou le maltose.

7. Préparation selon l'une quelconque des revendications 1 à 6, caractérisée par le fait que le surfactif est formé par: les mono- et triesters de polyoxyéthylènesorbitan; les alkylphénylpolyoxyéthylènes; le taurocholate de sodium, le cholate de sodium, le désoxycholate de sodium, le glycocholate de sodium; ou les polyalcools.

8. Préparation selon la revendication 7, caractérisée par le fait que l'ester de polyoxyéthylènesorbitan contient l'oléate, le laurate, le stéarate ou le palmitate d'acide gras.

9. Préparation selon l'une quelconque des revendications 1 à 8, caractérisée par le fait que la préparation doit être reconstituée avec de l'eau stérile.

10. Préparation selon l'une quelconque des revendications 1 à 7, caractérisée par le fait que la préparation contient, lors de la reconstitution avec de l'eau stérile: 1 à 16 mg/ml de fibronectine; est 0,002 M à 0,05 M en ce qui concerne un sel tampon physiologiquiment acceptable choisi entre le citrate de sodium, le glycinate de sodium, le phosphate de sodium ou le tris-(hydroxyméthyl)-aminométhane; est 0,03 M à 0,09 M en ce qui concerne le chlorure de sodium, contient 2 à 10% en poids/volume de glucose, de galactose, de mannose, de sucrose, de lactose, de maltose, de mannitol ou de sorbitol; et contient 0,01 à 0,4% en poids/volume d'un surfactif pharmaceutiquement acceptable choisi entre les mono- et triesters de polyoxyéthylènesorbitan; les alkylphénylpolyoxyéthylènes; le taurocholate de sodium, le cholate de sodium, le désoxycholate de sodium, le glycocholate de sodium; ou les polyalcools.

11. Préparation selon l'une quelconque des revendications 1 à 7, caractérisée par le fait que la préparation contient, lors de la reconstitution avec de l'eau stérile: 3 à 8 mg/ml de fibronectine; est 0,005 M à 0,02 M en ce qui concerne un sel physiologiquement acceptable choisi entre le citrate de sodium, le glycinate de sodium, le phosphate de sodium ou le tris-(hydroxyméthyl)-aminométhane; est 0,04 M à 0,08 M en ce qui concerne le chlorure de sodium, contient 3 à 7% en poids/volume de glucose, galactose, de mannose, de sucrose, de lactose, de maltose, de mannitol ou de sorbitol; et contient 0,02 à 0,1% en poids/volume d'un surfactif pharmaceutiquement acceptable choisi entre les mono- et triesters de polyoxyéthylènesorbitan; les alkylphénylpolyoxyéthylènes; le taurocholate de sodium, le cholate de sodium, le déoxycholate de sodium, le glycocholate de sodium; ou les polyalcools.

12. Préparation selon l'une quelconque des revendications 9 à 11, caractérisée par le fait que la préparation a un pH de 6,5 à 7,5.

13. Préparation selon la revendication 10, caractérisée par le fait que l'ester de polyoxyéthylènesorbitan contient l'oléate, le laurate, le stéarate et le palmitate d'acide gras.

14. Préparation selon la revendication 10, caractérisée par le fait que la fibronectine lyophilisée est reconstituée en une solution aqueuse en l'espace de 20 minutes.

**Revendications pour l'Etat Contractant: AT**

1. Procédé pour préparer une préparation de fibronectine lyophilisée en lyophilisant une solution aqueuse contenant 1 à 16 mg/ml de fibronectine et 0,01 à 0,4% en poids/volume d'un surfactif pharmaceutiquement acceptable, caractérisé par le fait que la solution contient en outre 2 à 10% en poids/volume d'un monosaccharide, d'un disaccharide, de mannitol ou de sorbitol.

2. Procédé selon la revendication 1, caractérisé par le fait que la solution est 0,03 M à 0,09 M en ce qui concerne un sel neutre.

3. Procédé selon la revendication 2, caractérisé par le fait que le sel neutre est le chlorure de sodium.

4. Procédé selon la revendication 1, caractérisé par le fait que la solution est 0,002 M à 0,05 M en ce qui concerne un sel tampon physiologiquement acceptable.

5. Procédé selon l'une quelconque des revendications 1 à 4, caractérisé par le fait que le sel tampon est le citrate de sodium, le glycinate de sodium, le phosphate de sodium, le tris-(hydroxyméthyl)-aminométhane ou des mélanges de ceux-ci.

6. Procédé selon l'une quelconque des revendications 1 à 5, caractérisé par le fait que le mono- ou disaccharide est le glucose, le galactose, le mannose, le sucrose, le lactose ou le maltose.

7. Procédé selon l'une quelconque des revendications 1 à 6, caractérisé par le fait que le surfactif est constitué par: les mono- et triesters de polyoxyéthylènesorbitan; les alkylphénylpolyoxyéthylène; le taurocholate de sodium, le cholate de sodium, le desoxycholate de sodium, le glycocholate de sodium; ou les polyalcools.

8. Procédé selon la revendication 7, caractérisé par le fait que l'ester de polyoxyéthylènesorbitan contient l'oléate, le laurate, le stéarate ou le palmitate d'acide gras.

9. Procédé selon l'une quelconque des revendications 1 à 8, caractérisé par le fait que l'on reconstitue la préparation avec de l'eau stérile.

10. Procédé selon l'une quelconque des revendications 1 à 7, caractérisé par le fait que la préparation contient, lors de la reconstitution avec de l'eau stérile: 1 à 16 mg/ml de fibronectine; est 0,002 M à 0,05 M ce qui concerne un sel tampon physiologiquement acceptable choisi entre le citrate de sodium, le glycinate de sodium, le phosphate de sodium ou le tris-(hydroxyméthyl)-aminométhane; est 0,03 M à 0,09 M en ce qui concerne le chlorure de sodium; contient 2 à 10% en poids/volume de glucose, de galactose, de mannose, de sucrose, de lactose, de maltose, de mannitol ou de sorbitol; et contient 0,01 à 0,4% en poids/volume d'un surfactif pharmaceutiquement acceptable choisi entre les mono- et triesters de polyoxyéthylènesorbitan; les alkylphénylpolyoxyéthylènes; le taurocholate de sodium, le cholate de sodium, le désoxycholate de sodium, le glycocholate de sodium; ou les polyalcools.

11. Procédé selon l'une quelconque des revendications 1 à 7, caractérisé par le fait que la préparation contient, lors de la reconstitution avec de l'eau stérile: 3 à 8 mg/ml de fibronectine; est 0,005 M à 0,02 M en ce qui concerne un sel physiologiquement acceptable choisi entre le citrate de sodium, le glycinate de sodium, le phosphate de sodium ou le tris-(hydroxyméthyl)-aminométhane; est 0,04 M à 0,08 M en ce qui concerne le chlorure de sodium; contient 3 à 7% en poids/volume de glucose, de galactose, de mannose, de sucrose, de lactose, de maltose, de mannitol ou de sorbitol; et contient 0,02 à 0,1% en poids/volume d'un surfactif pharmaceutiquement acceptable choisi entre les mono- et triesters de polyoxyéthylènesorbitan; les alkylphénylpolyoxyéthylènes; le taurocholate de sodium, le cholate de sodium, le désoxycholate de sodium, le glycocholate de sodium; ou les polyalcools.

12. Procédé selon l'une quelconque des revendications 9 à 11, caractérisé par le fait que la préparation a un pH de 6,5 à 7,5.

13. Procédé selon la revendication 10, caractérisé par le fait que l'ester de polyoxyéthylènesorbitan contient l'oléate, le laurate, le stéarate et le palmitate d'acide gras.

14. Procédé selon la revendication 10, caractérisé par le fait que l'on reconstitue la fibronectine lyophilisée en une solution aqueuse en l'espace de 20 minutes.